# EUROPEAN PATENT SPECIFICATION

(11) **EP 3 170 903 B1**
(45) Date of publication and mention of the grant of the patent: **18.09.2019**
(21) Application number: 15195682.8
(22) Date of filing: 20.11.2015
(51) Int. Cl.: C12Q 1/68, C12N 15/10

(54) **METHOD FOR PROCESSING A WATER-IN-OIL EMULSION**
VERFAHREN ZUR VERARBEITUNG EINER WASSER-IN-ÖL-EMULSION
PROCÉDÉ DE TRAITEMENT D'UNE ÉMULSION EAU DANS HUILE

(43) Date of publication of application: 24.05.2017
(73) Proprietor: Qiagen GmbH, 40724 Hilden (DE)
(72) Inventor: Wahl, Matthias, 42781 Haan (DE)
(74) Representative: Witte, Weller & Partner Patentanwälte mbB

(56) References cited:
- WO-A1-2010/014920
- WO-A2-2014/068407
- WO-A2-2015/170187
- SINGH AJAY ET AL: "Surfactants in microbiology and biotechnology: Part 2. Application aspects.", BIOTECHNOLOGY ADVANCES 2007 JAN-FEB, vol. 25, no. 1, January 2007 (2007-01), pages 99-121, XP005823204, ISSN: 0734-9750

## Description

The present invention is directed to a method for processing a water-in-oil-emulsion, for example in the context of or subsequent to an emulsion polymerase chain reaction (emPCR).

### FIELD OF THE INVENTION

The present invention relates to the field of molecular biology, more particularly to the amplification of nucleic acid molecules.

### BACKGROUND OF THE INVENTION

In the field of molecular or recombinant biology for many tasks it is necessary to immobilize a large number of DNA molecules on surfaces.

The polymerase chain reaction (PCR) is a technology in molecular biology used to amplify a single copy or a few copies of a piece of DNA across several orders of magnitude, generating thousands to millions of copies of a particular DNA sequence.

Recently, PCR-based methods have been adapted to amplifying molecules contained within water-in-oil emulsions. In such amplification methods a plurality of biological samples, e.g. nucleic acid samples, may be individually encapsulated in small aqueous micelles forming reaction compartments in the surrounding oil phase. PCR amplification is conducted on each of the plurality of encapsulated nucleic acid samples simultaneously. Such reaction compartments or microcapsules are often referred to as "microreactors" because the amplification occurs within the reaction compartments. The PCR is then often referred to as emulsion PCR (emPCR).

The small reaction compartments can include a template bead or microsphere and the amplification process may be referred to as bead-based emulsion PCR. In such a technique, beads along with DNA templates are suspended in an aqueous reaction mixture and then encapsulated in an inverse (water-in-oil) emulsion. The template DNA may be either bound to the bead prior to emulsification or may be included in solution in the amplification reaction mixture. For further details regarding techniques for bead emulsion amplification, reference is made to PCT publication WO 2005/073410 A2.

Emulsion PCR finds a widespread application in molecular diagnostics, such as in the he RainDrop® Digital PCR System, RainDance Technologies, Inc., Billerica, MA, U.S.A. Additionally, emulsion PCR is being employed for target amplification for several next generation sequencing (NGS) platforms, such as Roche/545, ThermoFisher Scientific Ion Torrent™, and the QIAGEN GeneReader.

Subsequent to an emPCR the oil phase is to be removed or the emulsion is to be "broken" in order to isolate the amplified nucleic acids from the reaction compartments. Such process is referred to as the "breaking step".

To remove the oil phase in the breaking step isopropanol [(CH₃)₂CHOH] and butanol [CH₃(CH₂)₃OH] are the reagents of choice. However, both have severe disadvantages, including light inflammability, acute toxicity, low flash point, causing corrosion, and unpleasant odor.

Prior art documents WO 2010/014920 and WO 2014/068407 disclose emulsion PCRs in which the breaking solution contains sodium lauryl sulfate or sodium dodecyl sulfate, respectively. However, these approaches in the art have not proved to provide good results.

Against this background, it is an object of the present invention to provide a new method for breaking a water-in-oil emulsion comprising a plurality of water droplets which include nucleic acid molecules.

The present invention satisfies these and other needs.

### SUMMARY OF THE INVENTION

The present invention provides a method for processing a water-in-oil emulsion, comprising the following steps:
1) providing a water-in-oil emulsion, said emulsion comprises a plurality of aqueous droplets, wherein at least a fraction of said aqueous droplets includes nucleic acid molecules; and
2) adding a solution comprising an anionic surfactant to said water-in-oil emulsion to obtain a 'broken solution', wherein said anionic surfactant is ammonium lauryl sulfate.

The inventor has developed a new method to break an water-in-oil emulsion, preferably in the context of or subsequent to an emulsion PCR, respectively. The method according to the invention may also be understood as a method for breaking such a water-in-oil emulsion. The method according to the invention has numerous advantages over the currently used method in the art.

The use of an anionic surfactant has numerous advantages over the use of isopropanol or butanol. It is hardly inflammable, non-toxic, doesn't smell, does not cause corrosion. Importantly, the anionic surfactant does not adversely affect the performance of amplified nucleic acid molecules and any downstream enzymatic reactions, such as the sequencing reaction mediated by a sequencing polymerase.

According to the invention an "anionic surfactant" refers to anionic compounds that lower the surface tension or interfacial tension between two liquids or between a liquid and a solid. Anionic surfactants contain anionic functional groups at their head, such as sulfate, sulfonate, phosphate, and carboxylates. Prominent alkyl sulfates include ammonium lauryl sulfate.

According to the invention a "solution" comprising said anionic surfactant refers to a solution which may contain the anionic surfactant dissolved in an appropriate carrier such as a buffer, e.g. TE buffer. It may also refer to a pure anionic surfactant solution.

According to the invention "aqueous droplets" refer to the aqueous phase of the water-in-oil emulsion consisting of a plurality of small micelles forming compartments in the surrounding oil phase capable of containing nucleic acid molecules. The aqueous droplets may be reaction compartments or microcapsules or "microreactors" for an emulsion PCR.

According to the invention at least a fraction of the aqueous droplets includes nucleic acid molecules. This means that there may be aqueous droplets without any nucleic acid molecules. However, it may also be the case that all of the aqueous droplets contain nucleic acid molecules. The nucleic acid molecules are in the following also referred to as "nucleic acid molecules of interest". In an embodiment of the invention such nucleic acid molecules may include a template nucleic acid molecule and copies thereof resulting from an amplification method, such as a polymerase chain reaction (PCR).

According to the invention the nucleic acid molecule may be of any nature, i.e. DNA, RNA, etc., whereas DNA is preferred.

After the addition of the solution comprising the anionic surfactant the emulsion is "broken" and a "broken solution" is obtained. Such step may include a resuspension or vigorously mixing, e.g. by pipetting (as done on the GeneRead QIAcube) or on a Vortex® device, to obtain a homogeneous solution. According to the invention, "breaking" means demulsifying or a demulsification of the water-in-oil emulsion. The breaking or demulsification leads to the complete or partial separation of the water-in-oil emulsion into oil and water layers.

According to the invention the anionic surfactant is ammonium lauryl sulfate.

This measure has the advantage that such kind of anionic surfactant is employed which, according to the findings of the inventor, yields especially good results.

In an embodiment of the invention said solution provides said ammonium lauryl sulfate in said water-in-oil emulsion in a final concentration of between approx. 1 - 50 wt.-%, preferably approx. 2 - 40 wt.-%, more preferably approx. 3 - 30 wt.-%, more preferably approx. 4 - 20 wt.-%, more preferably approx. 5 - 15 wt.-%, most preferably approx. 10 wt.-%.

This embodiment has the advantage that said ammonium lauryl sulfate is added to the water-in-oil emulsion in a concentration which ensures an effective breaking and removal of the oil phase. The skilled person can easily prepare a stock solution comprising the ammonium lauryl sulfate in the concentration needed in order to end up with the final concentrations as prescribed in this embodiment.

It is to be understood that the ammonium lauryl sulfate may be added to the emulsion in several steps where, possibly, different amounts or concentrations may be added. There may be a first step of adding the ammonium lauryl sulfate in a considerably high concentration and (a) following step(s) of adding the ammonium lauryl sulfate in a lower concentration, e.g. 10 wt.-% followed by 5 wt.-%, possibly with a washing steps in between.

In an embodiment of the invention said fraction of said aqueous droplets containing nucleic acid molecules includes one or more solid carriers, preferably micropheres, capable of capturing said nucleic acid molecules.

This measure has the advantage that the structural preconditions are established for using the invention in the context of or subsequent to a traditional emulsion PCR. "Capable of capturing" includes a state where the nucleic acid molecules are actually captured by the solid carriers. In an embodiment of the invention the capability can be established, e.g., by providing the solid carriers with nucleic acid molecules comprising a nucleotide sequence complementary to the nucleotide sequence of the nucleic acid molecule of interest. The nucleic acid molecule of interest can then be anchored to the solid carrier.

According to the invention a "solid carrier" refers to a physical entity allowing the adhesion of a nucleic acid. A preferred solid carrier is a microsphere which is traditionally used in an emulsion PCR. In the following the term "bead" is interchangeably used for "microsphere".

The inventor has surprisingly found out that the microspheres and the adhering nucleic acid molecules are not adversely affected by the ammonium lauryl sulfate.

Examples of suitable microspheres or beads are superparamagnetic spherical polymer particles ("magnetic beads"), for example Thermo Fischer Scientific Dynabeads® Magnetic Beads, polymer beads, for example polystyrene beads or polyacrylamide beads, silica beads, agarose beads.

The interaction between the solid carrier and nucleic acid molecules to capture the latter to the carrier may be mediated by covalent bonds, e.g. capture nucleic acid molecules are covalently bound to the solid carrier's surface, or by high noncovalent affinity interaction, e.g. between biotin and streptavidin.

According to a preferred embodiment in the method of the invention the following further step (3) is carried out:
3) separating said solid carriers together with captured nucleic acid molecules from said 'broken solution'.

This further step allows the recovering of the captured nucleic acid molecules by removing the solid carriers, e.g. microspheres, with the nucleic acid molecules attached thereto, from the solution. This separation step may include the washing of the solid carriers together with captured nucleic acid molecules to remove residual traces of the oil phase from the nucleic acid molecules.

According to a preferred embodiment in the method of the invention after step (2) and before step (3) the following further step (2.1) is carried out:
2.1) mixing the 'broken solution'.

This measure ensures an effective disintegration of the water-in-oil emulsion. The mixing can be realized by a resuspension, e.g. by pipetting (as done in the GeneRead QIAcube) or vigorously shaking, e.g. on a Vortex® device, to obtain a homogeneous solution.

According to an embodiment of the invention said microspheres comprise magnetic properties.

This measure has the advantage that a convenient and simple separation of the captured nucleic acids can be achieved by applying a magnetic field to the broken solution.

In a preferred embodiment of the method according to the invention said separation occurs by sedimenting said solid carriers together with captured nucleic acid molecules, preferably via centrifugation of said broken solution.

This measure takes advantage of a common separation technology for separating solid particles from a liquid phase.

In a preferred embodiment of the method according to the invention where the solid carrier comprises magnetic properties said separation occurs via the application of a magnetic field to said broken solution.

This measure incorporates a well-established technology for separating magnetic microspheres from a liquid phase.

In a further development of the method according to the invention the supernatant is removed and said nucleic acid molecules are recovered.

This step ensures the isolation of the nucleic acid molecules adhered to the solid carriers.

Said recovering may be realized via an enrichment of solid carriers capturing said nucleic acid molecules over such solid carriers not capturing said nucleic acid molecules. This enrichment step can be realized by filtering the solid carriers through a filter having pores with a size allowing a passing-through of solid carriers not capturing said nucleic acid molecules but a withhold of solid carriers capturing said nucleic acid molecules. Preferably said filtering is carried out after complexing solid carriers capturing said nucleic acid molecules with 'enrichment beads'. Such 'enrichment beads' may be provided with streptavidin, whereas the nucleic acids of interest captured by the solid carrier may be provided with a biotin modification. The complexing of the solid carriers capturing said nucleic acid molecules with 'enrichment beads' is then realized via a streptavidin-biotin interaction.

In another embodiment the method according is carried out subsequent to an emulsion polymerase chain reaction (emPCR).

This measure results in an improvement of the current emPCR by avoiding isopropanol and butanol. The breaking step in or subsequent to an emPCR as currently carried out in the art is the most critical step. Here, the invention provides effective remedy.

Another subject-matter of the invention is the use of an ammonium lauryl sulfate for breaking a water-in-oil emulsion, said emulsion comprises a plurality of water droplets, wherein at least a fraction of said water droplets include nucleic acid molecules.

The features, characteristics, advantages and embodiments specified for the method according to the invention apply likewise to the use according to the invention.

Another subject-matter of the present invention relates to a kit for carrying out an emulsion polymerase chain reaction (emPCR) with a nucleic acid molecule of interest comprising an ammonium lauryl sulfate for breaking a water-in-oil emulsion, a reaction vial and an experimental manual for carrying out the method according to the invention.

A kit is a combination of individual elements useful for carrying out the methods of the invention, wherein the elements are optimized for use together in the methods. The kits also contain additional reagents, chemicals, buffers, reaction vials etc. which may be useful for carrying out the method according to the invention. Such kits unify all essential elements required to work the method according to the invention, thus minimizing the risk of errors. Therefore, such kits also allow semi-skilled laboratory staff to perform the method according to the invention.

The features, characteristics, advantages and embodiments specified for the method according to the invention apply likewise to the kit according to the invention.

It is to be understood that the before-mentioned features and those to be mentioned in the following cannot only be used in the combination indicated in the respective case, but also in other combinations or in an isolated manner without departing from the scope of the invention as defined in the appended claims.

The invention is now further explained by means of embodiments resulting in additional features, characteristics and advantages of the invention. The embodiments are of pure illustrative nature and do not limit the scope or range of the invention. The features mentioned in the specific embodiments are general features of the invention which are not only applicable in the specific embodiment but also in an isolated manner in the context of any embodiment of the invention as defined in the appended claims.

The invention is now described and explained in further detail by referring to the following non-limiting examples and drawings.
- Fig. 1:: shows a flow chart illustrating an embodiment of the method according to the invention and the subsequent steps;
- Fig. 2:: shows a flow chart illustrating another embodiment of the method according to the invention and the subsequent enrichment of monoclonal beads via complexing them to 'enrichment beads';
- Fig. 3:: shows a comparison of an embodiment of the method according to the invention with the prior art method in respect of (A) sequencing performance, (B) enrichment, and (C) recovery.

### Examples

### 1. Method according to the invention

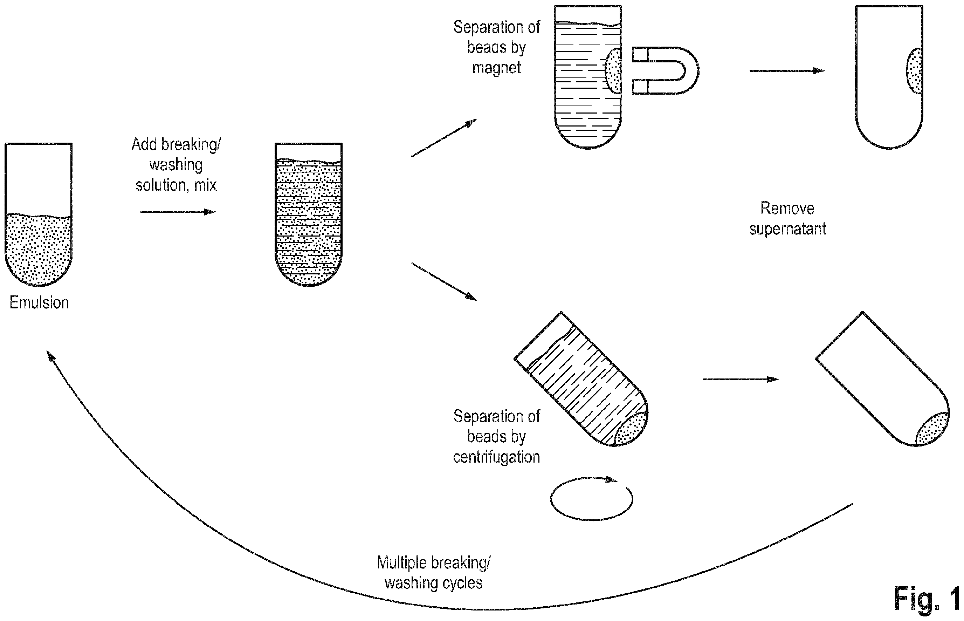

In Fig. 1 the method according to the invention and the subsequent steps are illustrated by means of an embodiment. An emulsion is provided where aqueous reaction compartments are separated by an oil phase. Those reaction compartments or droplets contain all reagents necessary for an PCR amplification and, ideally, one single library fragment. Usually, the PCR is carried out on the surface of microspheres or beads which capture the target and amplified nucleic acids. The PCR is also referred to as emulsion PCR (emPCR).

After the emPCR has been carried out buffer containing an anionic surfactant is added to the emulsion, e.g. 10 wt.-% ammonium lauryl sulfate (ALS) in the final concentration. The oil phase is then 'broken' by resuspending or vigorously mixing the emulsion after emPCR.

In case of using magnetic microspheres a magnetic field is applied to the broken solution resulting in the confining of the microspheres in the sphere of the magnetic field. The supernatant is removed. Alternatively or additionally the microspheres can be spun down by centrifuging the broken solution, followed by a removal of the supernatant.

The microspheres are then washed to remove residual traces of the oil phase by resuspending or vigorously mixing the beads with different wash buffers followed by the removal of the supernatant. Whereas the "breaking step" may consist of only one cycle of breaking with the anionic surfactant (e.g. 10 wt.-% ALS) multiple washing cycles may follow, e.g. by first using 2 wt.-% ALS in TE buffer and then TE buffer only.

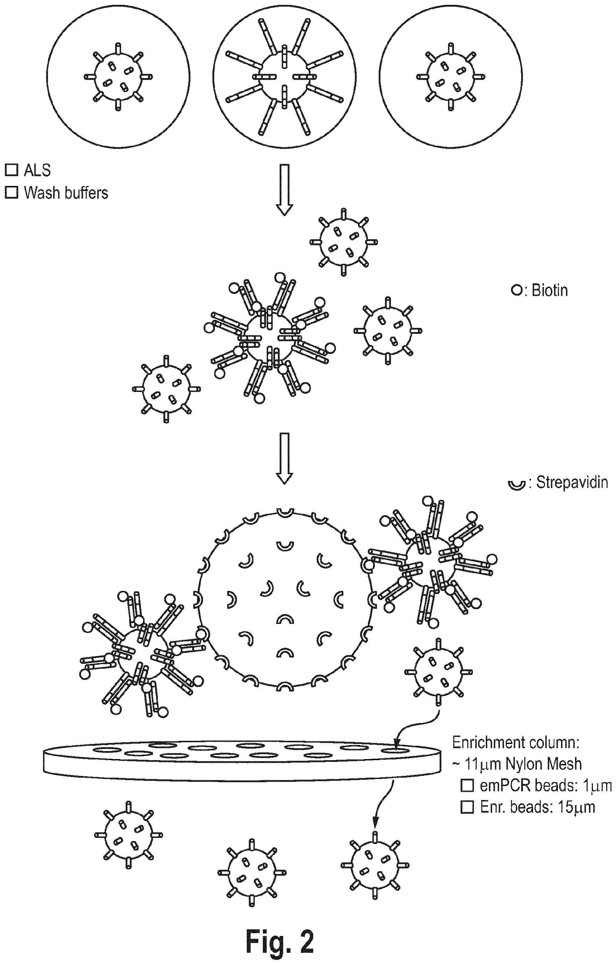

In Fig. 2 another embodiment of the method according to the invention is illustrated focusing of the subsequent enrichment steps.

One feature of the methodologies commonly used for emulsion making which include, among others, pipetting, stirring, porous membranes, or microfluidic devices, is that DNA or library fragments thereof, respectively, are statistically distributed over the droplets. In order to minimize droplets with multiple library fragments which would lead to non-clonally amplified libraries which cannot be sequenced, library fragments are diluted so that a large subset of droplets do not contain a library fragment.

In next generation sequencing platforms using emulsion PCR for clonal amplification of library fragments on sequencing beads, including IonTorrent and 454, this leads to a large subset of beads that do not contain any library fragment/DNA.

Those beads without DNA, so called "null beads", in turn would reduce the output of the subsequent sequencing reaction. Therefore, null beads are removed in a process called enrichment. The efficiency of the enrichment step is of high relevance as it helps maximizing the output of a NGS system by reducing non-informative beads w/o DNA.

In Fig. 2A a monoclonal bead in a droplet of a reaction compartment is shown next to a bead without an amplicon or a "null bead" in another reaction compartment. The reaction compartments are surrounded and separated by the oil phase.

In the subsequent breaking step an anionic surfactant is added to the emulsion to remove the oil phase and the beads are recovered.

Beads with an amplicon, so called "live beads", are enriched, beads without an amplicon ("null beads") are depleted. Enrichment beads with a diameter of -15 µm are used that specifically bind to live beads through a biotin-streptavidin interaction. Biotin is present on the reverse primer and will be only on beads if a PCR amplification has happened. Streptavidin is present on capture beads. The complexing of the live beads with the enrichment beads results in large complexes. The mixture is then subjected to an "enrichment column" having a nylon mesh with a pore size of ∼11 µm. The null beads can pass the enrichment column whereas the live beads complexed to the enrichments beads are withhold and, therefore, enriched due to the large size of the complexes.

The live beads are then released from the enrichment beads by denaturation, e.g. via treatment with NaOH.

### 2. Experiments demonstrating the effectivity of the invention

### 2.1 Material and methods

### Library/DNA template

A barcoded gene panel template was used as the sample for the evaluation. NGHS-101X Clinically Relevant Tumor Panel library was prepared from human control DNA NA12878 (Coriell Institute) using the standard GeneRead™ Library Preparation method. Libraries with barcodes were used in order to enable pooling of multiple samples so that they could be run in a single flowcell.

### Experimental procedure

Droplet making and emulsion PCR cycling was performed as described in the GeneRead™ Clonal Amp Q Kit and GeneRead™ QIAcube manuals. After emulsion PCR, emulsions were manually pooled into 50 ml tubes (i.e. the content of 48 wells which correspond to a sample).

Next, samples were split into two groups. The first group was broken twice (addition of breaking solution followed by thorough mixing and centrifugation/magnetic separation to collect beads) with isopropanol and the second group was broken twice with a 2:1 mix of 30% ammonium lauryl sulfate and TE (→ final ALS concentration: 10%).

After that initial breaking, the samples were manually processed using the GeneRead™ Clonal Amp Q Kit (i.e. performing the same steps that are normally conducted by the GeneRead™ QIAcube), with modifications (two additional wash steps and an treatment with exonuclease I). Finally, samples were subjected to a sequencing run on the GeneReader™ using the GeneRead™ Sequencing Q kit (using earlier internal versions which slightly differ from the launch configuration).

### QC assay for bead recovery: light scatter assay

To evaluate the performance of both alternative methods for breaking, intermediates were analyzed after breaking and enrichment using a light scatter assay. Light scattering of beads was measured at OD600 relative to a known standard in order to evaluate the recovery.

### QC assay for assessing enrichment efficiency based on flow cytometry (FACS)

Beads were hybridized with a fluorescent oligonucleotide that specifically binds beads with amplicon. In this way, the fraction of amplicon positive beads could be measured by flow cytometry.

### 2.2 Results

In order to assess the applicability of a 10 wt.-% ALS solution in breaking emulsions, an experiment was set up where a total of 8 independent emulsion PCR samples were broken with 10 wt.-% ALS and Isopropanol (prior art method), respectively (8 replicates for each condition; 16 in total). After breaking, all 16 samples were subjected to the GeneReader workflow (washing, enrichment and sequencing followed by an analysis of primary sequencing parameters).

### Sequencing performance

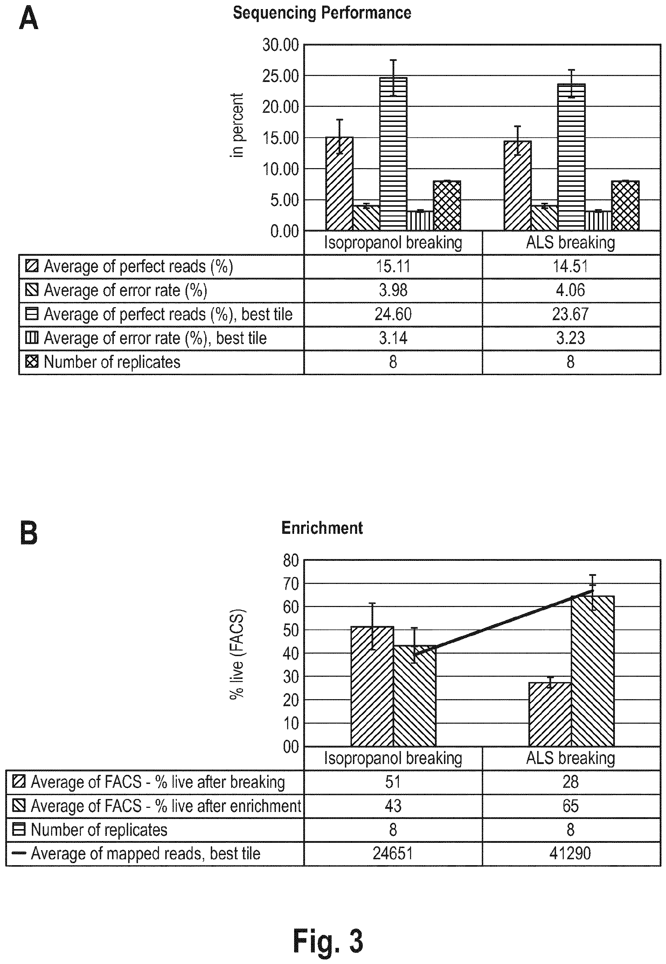

As shown in Fig. 3A, it results that beads or microspheres isolated from an emulsion which was broken with 10 wt.-% ALS show a sequencing performance similar to such obtained with beads/microspheres isolated from an emulsion which was broken by the prior art method.

### Enrichment

As depicted in Fig. 3B, it turns out that beads/microspheres isolated from an emulsion which was broken with 10 wt.-% ALS exhibit an enrichment factor which is better than that obtained with beads/microspheres isolated from an emulsion which was broken by the prior art method. The output is, therefore, better than with the prior art method.

### Recovery

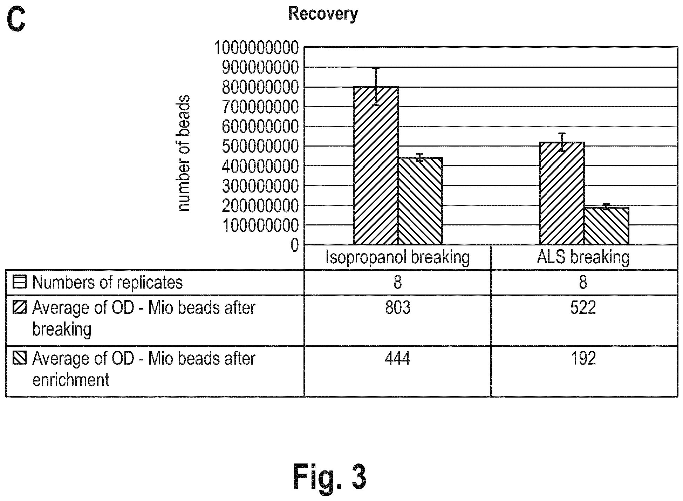

As demonstrated in Fig. 3C, beads/microspheres isolated from an emulsion which was broken with 10 wt.-% ALS show sufficient recovery after breaking and enrichment, i.e. less unspecific beads/microspheres.

## Claims

1. A method for processing a water-in-oil emulsion, comprising the following steps:
1) providing a water-in-oil emulsion, said emulsion comprises a plurality of aqueous droplets, wherein at least a fraction of said aqueous droplets includes nucleic acid molecules; and
2) adding a solution comprising an anionic surfactant to said water-in-oil emulsion to obtain a 'broken solution, wherein said anionic surfactant is ammonium lauryl sulfate.

2. The method of claim 1, **characterized in that** said solution provides said ammonium lauryl sulfate in said water-in-oil emulsion in a final concentration of between approx. 1 - 50 wt.-%, preferably approx. 2 - 40 wt.-%, more preferably approx. 3 - 30 wt.-%, more preferably approx. 4 - 20 wt.-%, more preferably approx. 5- 15 wt.-%, most preferably approx. 10 wt.-%.

3. The method of claim 1 or 2, **characterized in that** said fraction includes one or more solid carriers capable of capturing said nucleic acid molecules.

4. The method of claim 3, **characterized in that** the following further step (3) is carried out:
3) separating said solid carriers together with captured nucleic acid molecules from said 'broken solution'.

5. The method of claim 4, **characterized in that** after step (2) and before step (3) the following further step (2.1) is carried out:
2.1) mixing the 'broken solution'.

6. The method of any of claims 3-5, **characterized in that** said solid carriers are microspheres, preferably said microspheres comprise magnetic properties.

7. The method of any of claims 3-6, **characterized in that** said separation occurs by sedimenting said solid carriers together with captured nucleic acid molecules, preferably via a centrifugation of said 'broken solution'.

8. The method of claim 6 or 7, **characterized in that** said separation occurs via the application of a magnetic field to said 'broken solution'.

9. The method of claim 7 or 8, **characterized in that** the supernatant is removed and said nucleic acid molecules are recovered.

10. The method of claim 9, **characterized in that** said recovering is realized via an enrichment of solid carriers capturing said nucleic acid molecules over such solid carriers not capturing said nucleic acid molecules, preferably by filtering the solid carriers through a filter configured to allow a passing-through of such solid carriers not capturing said nucleic acid molecules but a withhold of solid carriers capturing said nucleic acid molecules, further preferably said filtering is carried out after complexing solid carriers capturing said nucleic acid molecules with 'enrichment beads'.

11. The method of any of claims 1-9, **characterized in that** it is carried out subsequent to an emulsion polymerase chain reaction (emPCR).

12. Use of an ammonium lauryl sulfate for breaking a water-in-oil emulsion, said emulsion comprises a plurality of water droplets, wherein at least a fraction of said water droplets include nucleic acid molecules.

13. Kit for carrying out an emulsion polymerase chain reaction (emPCR) with a nucleic acid molecule of interest comprising ammonium lauryl sulfate for breaking a water-in-oil emulsion, a reaction vial and an experimental manual, both for carrying out the method of any of claims 1-11.

## Patentansprüche

1. Verfahren zur Bearbeitung einer Wasser-in-ÖI-Emulsion, das die folgenden Schritte aufweist:
1) Bereitstellung einer Wasser-in-ÖI-Emulsion, wobei die Emulsion eine Vielzahl von wässrigen Tropfen aufweist, wobei zumindest eine Fraktion der wässrigen Tropfen Nukleinsäuremoleküle beinhaltet; und
2) Zugeben von einer Lösung, die ein anionisches Surfactant aufweist, zu der Wasser-in-ÖI-Emulsion, um eine 'aufgebrochene Lösung' zu erhalten, wobei das anionische Surfactant Ammoniumlaurylsulfat ist.

2. Verfahren nach Anspruch 1, **dadurch gekennzeichnet, dass** die Lösung das Ammoniumlaurylsulfat in der Wasser-in-ÖI-Emulsion in einer Endkonzentration bereitstellt, die bei ungefähr 1 - 50 Gew.-%, vorzugsweise ungefähr 2 - 40 Gew.-%, weiter vorzugsweise ungefähr 3 - 30 Gew.-%, weiter vorzugsweise ungefähr 4 - 20 Gew.-%, weiter vorzugsweise ungefähr 5 - 15 Gew.-%, und höchst vorzugsweise ungefähr 10 Gew.-% liegt.

3. Verfahren gemäß Anspruch 1 oder 2, **dadurch gekennzeichnet, dass** die Fraktion einen oder mehrere feste Träger beinhaltet, die in der Lage sind, die Nukleinsäuremoleküle einzufangen.

4. Verfahren nach Anspruch 3, **dadurch gekennzeichnet, dass** der folgende weitere Schritt (3) ausgeführt wird:
3) Trennen der festen Träger zusammen mit eingefangenen Nukleinsäuremolekülen von der 'aufgebrochenen Lösung'.

5. Verfahren nach Anspruch 4, **dadurch gekennzeichnet, dass** nach Schritt 2) und vor Schritt 3) der folgende weitere Schritt (2.1) ausgeführt wird:
2.1) Mischen der 'aufgebrochenen Lösung'.

6. Verfahren nach einem der Ansprüche 3-5, **dadurch gekennzeichnet, dass** die festen Träger Mikrosphären sind, wobei die Mikrosphären vorzugsweise magnetische Eigenschaften aufweisen.

7. Verfahren nach einem der Ansprüche 3-6, **dadurch gekennzeichnet, dass** die Trennung durch Sedimentieren der festen Träger zusammen mit eingefangenen Nukleinsäuremolekülen erfolgt, vorzugsweise über eine Zentrifugation der'aufgebrochenen Lösung'.

8. Verfahren nach Anspruch 6 oder 7, **dadurch gekennzeichnet, dass** die Trennung über das Anliegen eines Magnetfeldes an die 'aufgebrochene Lösung' erfolgt.

9. Verfahren nach Anspruch 7 oder 8, **dadurch gekennzeichnet, dass** der Überstand entfernt und die Nukleinsäuremoleküle erhalten werden.

10. Verfahren nach Anspruch 9, **dadurch gekennzeichnet, dass** das Erhalten über eine Anreicherung der festen Träger, die die Nukleinsäuremoleküle einfangen, gegenüber solchen festen Trägern erfolgt, die die Nukleinsäuremoleküle nicht einfangen, vorzugsweise durch Filtern der festen Träger durch einen Filter, der ausgebildet ist, um ein Hindurchtretenlassen von solchen festen Trägern zu erlauben, die die Nukleinsäuremoleküle nicht einfangen, jedoch feste Träger, die die Nukleinsäuremoleküle einfangen, zurückhalten, wobei weiter vorzugsweise das Filtrieren nach dem Komplexieren der festen Träger, die die Nukleinsäuremoleküle einfangen, mit 'Anreicherungskugeln' durchgeführt wird.

11. Verfahren nach einem der Ansprüche 1-9, **dadurch gekennzeichnet, dass** es im Anschluss an eine Emulsionspolymerasekettenreaktion (emPCR) durchgeführt wird.

12. Verwendung eines Ammoniumlaurylsulfats zum Aufbrechen einer Wasser-in-ÖI-Emulsion, wobei die Emulsion eine Vielzahl von Wassertropfen aufweist, wobei zumindest eine Fraktion der Wassertropfen Nukleinsäuremoleküle beinhaltet.

13. Kit zur Durchführung einer Emulsionspolymerasekettenreaktion (emPCR) mit einem Nukleinsäuremolekül von Interesse, das Folgendes aufweist, nämlich Ammoniumlaurylsulfat zum Aufbrechen einer Wasser-in-ÖI-Emulsion, ein Reaktionsgefäß und eine experimentelle Gebrauchsanweisung, beides zum Durchführen des Verfahrens nach einem der Ansprüche 1 bis 11.

## Revendications

1. Procédé de traitement d'une émulsion eau dans l'huile, comprenant les étapes suivantes :
1) fournir une émulsion eau dans l'huile, ladite émulsion comprend une pluralité de gouttelettes aqueuses, où au moins une fraction desdites gouttelettes aqueuses comporte des molécules d'acide nucléique ; et
2) ajouter une solution comprenant un tensioactif anionique à ladite émulsion eau dans l'huile pour obtenir une « solution rompue », où ledit tensioactif anionique est le laurylsulfate d'ammonium.

2. Procédé de la revendication 1, **caractérisé en ce que** ladite solution fournit ledit laurylsulfate d'ammonium dans ladite émulsion eau dans l'huile à une concentration finale comprise entre environ 1 et 50% en poids, de préférence entre environ 2 et 40% en poids, plus préférablement entre environ 3 et 30% en poids, plus préférablement entre environ 4 et 20% en poids, plus préférablement entre environ 5 et 15% en poids, idéalement d'environ 10% en poids.

3. Procédé de la revendication 1 ou 2, **caractérisé en ce que** ladite fraction comporte un ou plusieurs support(s) solide(s) capable(s) de capturer lesdites molécules d'acide nucléique.

4. Procédé de la revendication 3, **caractérisé en ce que** l'étape supplémentaire suivante (3) est réalisée :
3) séparer desdits supports solides conjointement avec des molécules d'acide nucléique capturées de ladite « solution rompue ».

5. Procédé de la revendication 4, **caractérisé en ce qu'**après l'étape (2) et avant l'étape (3), l'étape supplémentaire suivante (2.1) est réalisée :
2.1) mélanger la « solution rompue ».

6. Procédé de l'une quelconque des revendications 3 à 5, **caractérisé en ce que** lesdits supports solides sont des microsphères, de préférence lesdites microsphères comprennent des propriétés magnétiques.

7. Procédé de l'une quelconque des revendications 3 à 6, **caractérisé en ce que** ladite séparation se produit par sédimentation desdits supports solides conjointement avec des molécules d'acide nucléique capturées, de préférence par une centrifugation de ladite « solution rompue ».

8. Procédé de la revendication 6 ou 7, **caractérisé en ce que** ladite séparation se produit par l'application d'un champ magnétique à ladite « solution rompue ».

9. Procédé de la revendication 7 ou 8, **caractérisé en ce que** le surnageant est éliminé et lesdites molécules d'acide nucléique sont récupérées.

10. Procédé de la revendication 9, **caractérisé en ce que** ladite récupération est réalisée par enrichissement de supports solides capturant lesdites molécules d'acide nucléique par rapport à de tels supports solides ne capturant pas lesdites molécules d'acide nucléique, de préférence par filtration des supports solides à travers un filtre configuré pour permettre un passage de tels supports solides ne capturant pas lesdites molécules d'acide nucléique mais une retenue de supports solides capturant lesdites molécules d'acide nucléique, en outre de préférence ladite filtration est réalisée après formation de complexes de supports solides capturant lesdites molécules d'acide nucléique avec des « billes d'enrichissement ».

11. Procédé de l'une quelconque des revendications 1 à 9, **caractérisé en ce qu'**il est réalisé à la suite d'une réaction en chaîne par polymérase en émulsion (emPCR).

12. Utilisation d'un laurylsulfate d'ammonium pour rompre une émulsion eau dans l'huile, ladite émulsion comprend une pluralité de gouttelettes d'eau, où au moins une fraction desdites gouttelettes d'eau comporte des molécules d'acide nucléique.

13. Kit pour réaliser une réaction en chaîne par polymérase en émulsion (emPCR) avec une molécule d'acide nucléique d'intérêt comprenant du laurylsulfate d'ammonium pour rompre une émulsion eau dans l'huile, un flacon de réaction et un manuel expérimental, permettant tous deux de réaliser le procédé de l'une quelconque des revendications 1 à 11.
